# EUROPÄISCHE PATENTSCHRIFT

(11) **EP 0 540 572 B1**
(45) Veröffentlichungstag und Bekanntmachung des Hinweises auf die Patenterteilung: **11.12.1996**
(21) Anmeldenummer: 91913016.1
(22) Anmeldetag: 19.07.1991
(51) Int. Cl.: C07D 405/12, C09K 19/58, C09K 19/34

(54) **CHIRALE ALKEN-ARYL-2,3-EPOXYALKYL-ETHER UND IHRE VERWENDUNG IN FLÜSSIGKRISTALLINEN MISCHUNGEN**
CHIRAL ALKEN-ARYL-2,3-EPOXYALKYL-ETHERS AND THEIR USE IN LIQUID CRYSTAL MIXTURES
ALCEN-ARYL-2,3-EPOXYALKYLETHERS CHIRAUX ET LEUR UTILISATION DANS DES MELANGES DE CRISTAUX LIQUIDES

(30) Priorität: 20.07.1990 DE 4023027
(43) Veröffentlichungstag der Anmeldung: 12.05.1993
(73) Patentinhaber: HOECHST AKTIENGESELLSCHAFT, 65926 Frankfurt am Main (DE)
(72) Erfinder: BLATTER, Karsten, D-6500 Mainz (DE); ESCHER, Claus, D-6109 Mühltal (DE); HARADA, Takamasa 4-35-15, Kikari Inzai-machi, Chiba (JP); HARNISCHFEGER, Peter, D-6100 Darmstadt (DE); HEMMERLING, Wolfgang, D-6231 Sulzbach (DE)
(86) Internationale Anmeldenummer: EP9101365
(87) Internationale Veröffentlichungsnummer: WO9201685

(56) Entgegenhaltungen:
- EP-A- 0 263 437
- EP-A- 0 292 954
- EP-A- 0 355 830

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu langsam. Dieser Nachteil macht sich insbesondere dann bemerkbar, wenn - was bei größeren Anzeigeelementflächen zwangsläufig der Fall ist - eine große Anzahl von Bildpunkten angesteuert werden muß, wodurch die Produktionskosten solcher, diese größeren Flächen enthaltenden Geräte wie Videogeräte, Oszillographen oder TV-, Radar-, EDV- oder Schreibautomaten-Bildschirme zu hoch wären.

Neben den nematischen und cholesterischen Flüssigkristallen haben seit einigen wenigen Jahren in zunehmendem Maße auch für Praxisanwendungen geneigt(tilted)-smektische Flüssigkristall-Phasen an Bedeutung gewonnen. Wenn solchen geneigt-smektischen Phasen, insbesondere smektisch C (S_{c} oder SmC)Phasen, geeignete Dotierstoffe zugesetzt werden, die eine sogenannte spontane Polarisation (Pₛ) zeigen oder in der Flüssigkristall-Phase induzieren, so können die Phasen in eine ferroelektrische Flüssigkristall-Phase umgewandelt werden (Benennung von Pₛ in nC·cm⁻²) siehe dazu beispielsweise Lagerwall et al. im Aufsatz "Ferroelectric Liquid Cristals for Displays" (Ferroelektrische Flüssigkristalle für Anzeigeelemente), SID Symposium, October Meeting, 1985, San Diego (USA). Diese ferroelektrischen Flüssigkristall-Phasen weisen - verglichen mit den üblichen TN("twisted nematic")-Zellen - um etwa den Faktor 1000 schnellere Schaltzeiten auf, so daß sie - auch aufgrund anderer positiver Eigenschaften wie einer bistabilen Schaltmöglichkeit - gute potentielle Kandidaten für die oben aufgeführten Anwendungsgebiete (z.B. über eine Matrixansteuerung) sind.

Auf der 11. Internationalen, Flüssigkristallkonferenz (30.6. bis 4.7.1986) in Berkeley/USA wurden von D.M. Walba ferroelektrische Flüssigkristalle vorgestellt, die chirale 2,3-Epoxy-alkyl-Seitenketten enthalten und folgende allgemeine Formel aufweisen:

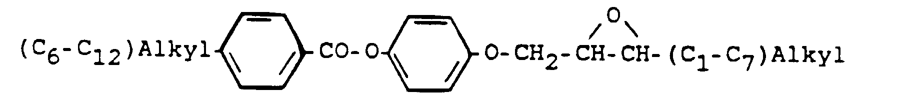

Die Verbindung mit einem C₁₀-Alkyl bzw. C₃-Alkylrest weist zwischen 75 °C und 80 °C eine SmC∗-Phase auf; die Schaltzeit (75 °C, 15 V/µm) beträgt 14 µsec, der Wert für die spontane Polarisation Pₛ beträgt 45 nC/cm².

In DE-A 36 33 968 wurden Verbindungen der allgemeinen Fomel (I) beschrieben 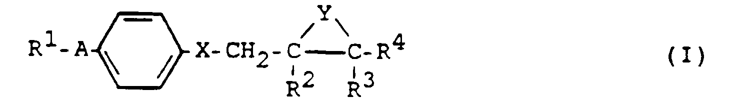 wobei
die Symbole folgende Bedeutung haben:
- R¹ =: geradkettiges oder verzweigtes (C₁-C₁₂)Alkyl, wobei eine oder zwei nicht-benachbarte CH₂-Gruppen durch O-und/oder S-Atome ersetzt sein können,
- A =: Diazin-2,5-diyl oder Diazin-3,6-diyl,
- X, Y =: O und/oder S, und
- R², R³, R⁴ =: unabhängig voneinander H, geradkettiges (C₁-C₁₀)Alkyl oder verzweigtes (C₃-C₁₀)Alkyl, wobei R²,R³ und R⁴ nicht gleichzeitig H sind.

Diese Verbindungen induzieren eine hohe spontane Polarisation in flüssigkristallinen Mischungen.

Aufgabe der vorliegenden Erfindung ist es, ungesättigte Verbindungen aufzuzeigen, die die Phasenbreite in LC-Mischungen erhöhen, den Schmelzpunkt der Mischungen erniedrigen, die bei hohen Werten für die eigene oder in Flüssigkristall-Phasen induzierte spontane Polarisation Pₛ Strukturelemente aufweisen, die sie auch mit anderen Komponenten in Flüssigkristall-Systemen "verträglich" (d.h. mischbar) machen, da u.a. der mesogene Teil der Moleküle oftmals für eine gute "Verträglichkeit" mit den anderen Mischungskomponenten in Flüssigkristall-Systemen verantwortlich ist; dabei müssen diese Verbindungen selbst nicht unbedingt flüssigkristallin sein, insbesondere nicht unbedingt eine SmC-Phase aufweisen.

Die Erfindung geht aus von den bekannten chiralen Verbindungen mit einem mesogenen aromatischen Baustein und einem chiralen Baustein mit einem heterocyclischen Dreiring.

Die erfindungsgemäßen Verbindungen sind dann dadurch gekennzeichnet, daß in der allgemeinen Formel (I) 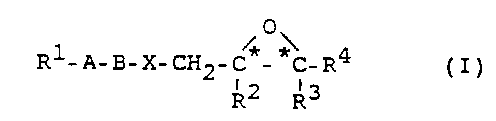 die Symbole folgende Bedeutung haben:
- R¹ =: geradkettiges oder verzweigtes (C₁-C₁₂)-(C₃-C₁₂)Alkenyl mit terminaler Doppelbindung, wobei eine oder zwei nicht-benachbarte CH₂-Gruppen durch 0- und/oder S-Atome ersetzt sein können,
- A, B =: unabhängig voneinander.Phenyl, Diazin-2,5-diyl oder Diazin-3,6-diyl, mit der Maßgabe, daß eine der Gruppen A oder B gleich Phenyl und die andere gleich Diazin ist,
- X =: O oder S
- R²,R³,R⁴ =: unabhängig voneinander H, geradkettiges (C₁-C₁₀)Alkyl oder verzweigtes (C₃-C₁₀)Alkyl, wobei R², R³ und R⁴ nicht gleichzeitig H sind.

Die N-Atome im Diazin-Ringsystem können sich dabei in 1,3-(Pyrimidine) oder in 1,2-Stellung (Pyridazine) befinden.

Unter den Verbindungen der allgemeinen Formel (I) sind die bevorzugt, bei denen die Symbole folgende Bedeutung haben: R¹ = geradkettiges (C₅-C₁₁)Alkenyl, wobei eine CH₂-Gruppe durch ein O- oder S-Atom ersetzt sein kann, X = O, R², R³ = H und R⁴ = geradkettiges oder verzweigtes (C₃-C₇)Alkyl.

Die Verbindungen der allgemeinen Formel (I) können beispielsweise hergestellt werden durch Umsetzung der Phenole oder Thiophenole der allgemeinen Formeln (II), (III) oder (IV) mit den Oxiranen der allgemeinen Formel (V); worin Z = H ist oder für eine nucleofuge Gruppe steht: 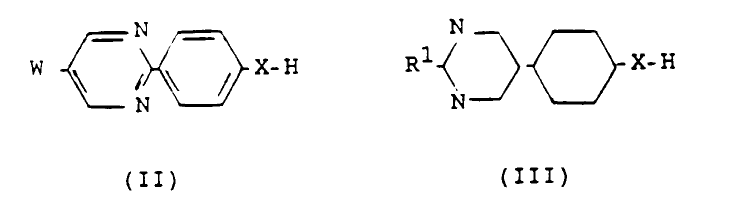 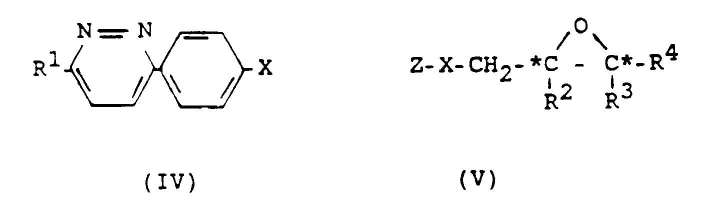
- W =: R¹, Benzyloxy, tert.-Butyldimethylsilyloxy, tert.-Butyldiphenylsilyloxy.

Die Verknüpfung der mesogenen Phenole oder Thiophenole der allgemeinen Formeln (II), (III) oder (IV) mit den chiralen Oxiranen der allgemeinen Formel (V) kann in an sich bekannter Weise erfolgen, z.B. durch Umsetzung von (II), (III) oder (IV) mit den Epoxyalkoholen (X=O und Z=H) unter Zuhilfenahme von Diethylazodicarboxylat und Triphenylphosphin, wie von Mitsonobu, "The Use of Diethyl Azodicarboxylate and Triphenylphosphine in Synthesis and Transformation Natural Products" in Synthesis 1981, S.1-28 beschrieben. Möglich ist es auch, die Epoxyalkohole mit einer derartigen nucleofugen Gruppe Z zu versehen, daß die Umsetzung mit Alkali oder Erdalkali-Salzen der Verbindungen (II), (III) oder (IV) zur Bildung der gewünschten Ether der allgemeinen Formel I (mit X=O) führt. Als derartige nucleofuge (Abgangs-)Gruppen sind u.a. Tosylate, Brosylate, Mesilate oder Triflate dem Fachmann bekannt, sie lassen sich in bekannter Weise z.B. aus den Alkoholen und den jeweiligen Säurehalogeniden herstellen.

Die Verknüpfung des Mesogens (W = Benzyloxy, tert.-Butyldimethylsilyloxy bzw.
tert.-Butyldiphenylsilyloxy) mit dem Epoxyalkohol kann jedoch auch als erster Schritt der Synthese erfolgen. Die Benzyl-Schutzgruppe kann durch katalytische Hydrierung in THF in Gegenwart von Palladium auf Aktivkohle entfernt werden, die Silyl-Schutzgruppen durch Behandlung mit Tetrabutylammoniumfluorid in THF. Im letzten Schritt kann das entstandene Phenol mit 1,n-Alkenolen nach MItsunobu umgesetzt werden. Die Reaktion der Alkali- oder Erdalkalisalze dieses Phenols mit l,n-T-Alkenen ist gleichfalls möglich, wobei T eine nucleofuge Gruppe wie z.B. Halogenid, Tosylat, Brosylat, Mesylat oder Triflat darstellt.

Die Edukte sind aus der Literatur bekannte Verbindungen. Beispielsweise können die Verbindungen (II) mit X=O durch Kondensation von substituierten Benzamiden mit 2-Alkylmalonsäureestern, Überführung der entstehenden Dihydroxypyrimidine in Dichlorpyrimidine und nachfolgende Hydrogenolyse hergestellt werden (siehe DE-C 22 57 588). Die Verbindungen (III) mit X=O werden beispielsweise durch Kondensation entsprechend substituierter 2-Aryl-3-(methylthio)acroleine mit geeigneten Amidinen hergestellt [siehe Kano et al., "A New and Facile Synthesis of 5-Arylpyrimidines and 4-Arylpyrazoles" in Heterocycles, Vol. 19, No. 6, 1079 bis 1082 (1982)]. Aus den Phenolen werden die entsprechenden Thiophenole nach bekannten Methoden erhalten [z.B. Newman et al., "The Conversion of Phenols to Thiphenoles via Dialkylcarbamates" in J. Org. Chem., 31, S. 3980-3984 (1966)].

Die Oxirane (Epoxyalkohole) als bevorzugte Verbindungen der allgemeinen Formel (V) mit X = O und Z = H werden beispielsweise aus den entsprechenden Allylalkoholen durch enantioselektive Epoxidierung hergestellt (siehe Pfenninger, "Asymmetric Epoxidation of Allylic Alcohols: The Sharpless Epoxidation" in Synthesis 1986, S. 89-116). Sie werden dann als solche eingesetzt (Z=H) oder aber nach Standardmethoden, z.B. durch Umsetzung mit 4-Toluolsulfonylchlorid, in die entsprechenden Tosylate überführt (Z=SO₂C₆H₄CH₃), analoges gilt für die anderen genannten nucleofugen Gruppen.

Eine weitere Lösung der gestellten Aufgabe ist eine verdrillbare Flüssigkristall-Phase mit einem Gehalt an mindestens einer chiralen Verbindung, die als chirale Verbindung mindestens eine Verbindung der allgemeinen Formel (I), enthält. Unter dem Begriff "verdrillbare Flüssigkristall-Phase" sind nematische, cholesterische oder geneigt("tilted")-smektische, insbesondere SmC-Phasen zu verstehen.

Die verdrillbaren Flüssigkristall-Phasen bestehen aus 2 bis 20, vorzugsweise 2 bis 15 Komponenten, darunter mindestens einer der erfindungsgemäß beanspruchten chiralen Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe der chiralen Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [=mindestens eine enantiotrope (Klärtemperatur > Schmelztemperatur) oder monotrope (Klärtemperatur < Schmelztemperatur) Mesophasenbildung erwarten läßt].

Insbesondere enthält die verdrillbare Flüssigkristall-Phase neben mindestens einer der erfindungsgemäß beanspruchten chiralen Verbindungen eine Phenylpyrimidinverbindung mit S_{c}-Phase, z.B. ein 4-(5-Alkyl-pyrimidin-2-yl)-1-alkoxybenzol.

In flüssigkristallinen Mischungen erzeugen die erfindungsgemäßen Verbindungen insbesondere eine Erniedrigung des Schmelzpunktes und eine Verbreitung des Temperaturbereichs der smektischen Phase.

Von der oder den erfindungsgemäßen Verbindung(en) enthalten die Flüssigkristall-Mischungen im allgemeinen 0,01 bis 70 Gew.-%, insbesondere 0,05 bis 50 Gew.-%.

Die erfindungsgemäßen Verbindungen sind insbesondere als Dotierstoffe für geneigt-smektische Flüssigkristall-Phasen geeignet, da sie diese in ferroelektrische Flüssigkristall-Phasen umwandeln.

Die Erfindung wird durch die nachstehenden Beispiele näher ausgeführt.

### Beispiele

### Beispiel 1

Herstellung von : 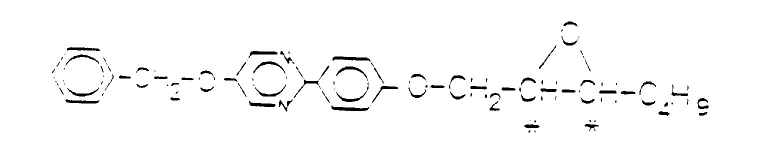 36,72 g (140 mmol) Triphenylphosphin werden in 350 ml Tetrahydrofuran gelöst und bei 0 °C mit 24,38 g (140 mmol = 21,77 ml) Diethylazodicarboxylat versetzt. Man rührt 20 min bei 0 °C, entfernt die Kühlung und gibt nacheinander 38,96 g (140 mmol) 5-Benzyloxy-2-(4-hydroxyphenyl)pyrimidin in 170 ml Tetrahydrofuran sowie 18,23 g (140 mmol) trans-(2S,3S)-3-Butyl-2-oxiranmethanol in 140 ml Tetrahydrofuran zu der Reaktionslösung, die 48 h bei Raumtemperatur gerührt wird. Man engt ein und reinigt das Rohprodukt durch Chromatographie an Kieselgel mit Dichlormethan/Ethylacetat (15:1). 19.04 g (35 %) Fp 139,5 141 °C.

### Beispiel 2

Herstellung von: 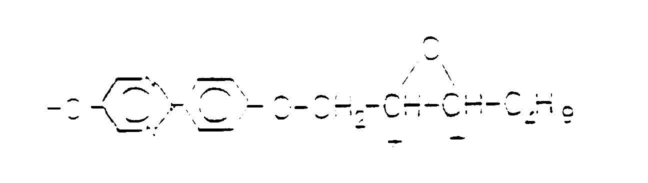 19,0 g (48,8 mmol) (2S,3S)-3-Butyl-2-oxiranmethyl[4-(5-benzyloxypyrimidin-2-yl)phenyl]ether in 500 ml Tetrahydrofuran werden in der Schüttelente in Gegenwart von 1,5 g 10 %igem Palladium auf Aktivkohle und 200 mg p-Toluolsulfonsäure mit Wasserstoff bei Atmosphärendruck hydriert. Man filtriert die Reaktionslösung über Coriolite, wäscht mit gesättigter Bicarbonatlösung sowie mit Kochsalzlösung, trocknet über Magnesiumsulfat und engt ein. Nach Chromatographie an Kieselgel mit Dichlormethan/ Ethylacetat (4:1) erhält man 10,89 g 2-[4-((2S,3S)-3-Butyl-2-oxiranmethyloxy)phenyl]-5-hydroxypyrimidin.

### Beispiel 3

Allgemeine Arbeitsvorschrift für die Herstellung von Verbindungen der Formel: 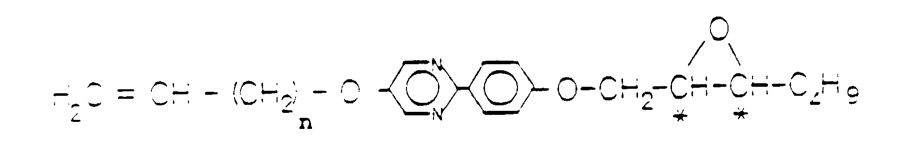 4,97 g (17 mmol) 2-[4-((2S,3S)-3-Butyl-2-oxiranmethyloxy) phenyl]-5-hydroxypyrimidin, 4,64 g (34 mmol) und a) 3,58 g (22 mmol) 6-Brom-1-hexan bzw. b) 4,20 g (22 mmol) 8-Brom-1-octen, bzw. c) 4,74 g (22 mmol) 10-Brom-1-decen bzw. d) 2,66 g (22 mmol) 3-Brom-1-propen werden in 60 ml Methylethylketon 24-48 h unter Rückfluß in 100 ml Chloroform/100 ml Wasser aufgenommen, die wässrige Phase mit 50 ml Chloroform nachextrahiert, die vereinigten organischen Phasen mit Wasser gewaschen und über Magnesiumsulfat getrocknet. Das Lösungsmittel wird entfernt und der Rückstand 2 x aus heißem Ethanol umkristallisiert.

### Man erhält:

a) 3,93 g (60,3 %) 5-(1-Hexan-6-yl)oxy-2-[4-((2S,3S)-3-butyl-2-oxiran)methyloxy)phenyl)]pyrimidin (1)
b) 4,12 g (60 %) 5-(1-Octen-8-yl)oxy-2-[4-((2S,3S)-3-butyl-2-oxiran)methyloxy)phenyl)]pyrimidin (2)
c) 4,53 g (62,3 %) 5-(1-Decen-10-yl)oxy-2-[4-((2S,3S)-3-butyl-2-oxiran)methyloxy)phenyl)]pyrimidin (3)
d) 3,25 g (56,3 %) 5-(2-Propen-3-yl)oxy-2-[4-((25,3S)-3-butyl-2-oxiran)methyloxy)]pyrimidin (4)

### Phasenfolgen:

1: X 78 N* 85 I
2: X 73 S_{C}* 85 S_{A}* 89 I
3: X 59 S_{C}* 93 S_{A}* 96 I
4: X 93 1

### Anwendungsbeispiel

A. Es wird eine Mischung aus folgenden 6 Komponenten hergestellt (in Mol.-%) 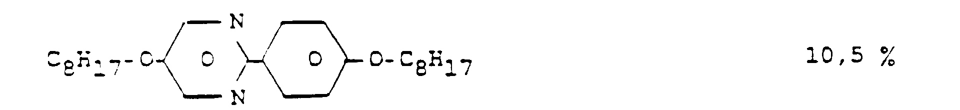 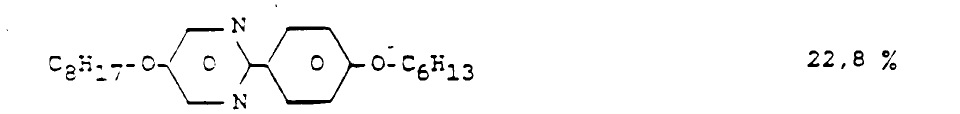 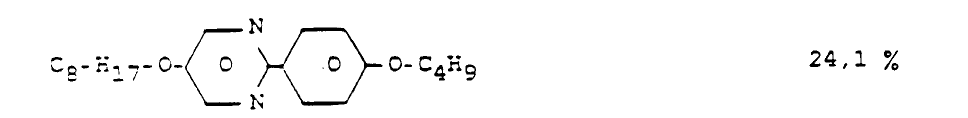 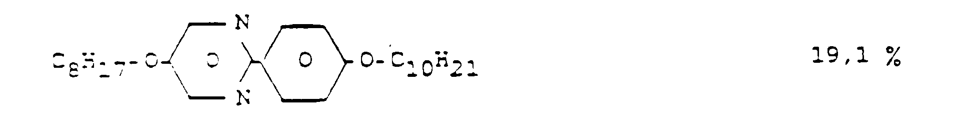 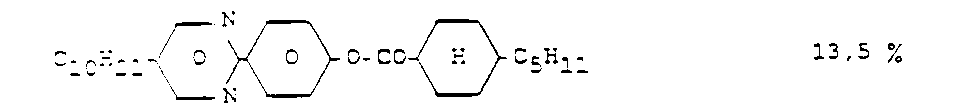 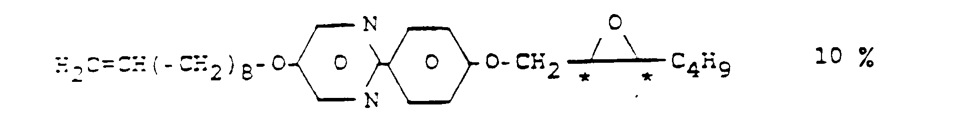 Anschließend wird die Polarisation und die Schaltzeit bei einer Temperatur von 25 °C gemessen:

Pₛ = 9 µC/cm²

tₛ = 200 µs

Die Mischung ist in einem Temperaturintervall von 20 °C bis 75 °C schaltbar.

Die Werte der spontanen Polarisation wurden nach der Methode von H. Diamant et al., Rev. Sci. Instr. 28, 30, 1957 bestimmt (2 µm Meßzelle, geriebenes Polyimid).
Die Schaltzeit wurde wie in DE-A-39 09 355 bestimmt.

B. Die folgenden beiden Beispiele zeigen die vorteilhafte Verwendung der erfindungsgemäßen Verbindungen im Vergleich zu analogen Verbindungen mit vollständig hydrierten Seitenketten.

Der Vorteil besteht in der vergleichsweise höheren Schaltgeschwindigkeit bzw. äquivalent in der bei gleicher Schaltgeschwindigkeit geringeren zum Schalten notwendigen Spannung. Dies ist teilweise auf einen kleineren induzierten Schaltwinkel und teilweise auf eine geringere Viskosität zurückzuführen.

### Beispiel (erfindungsgemäß)

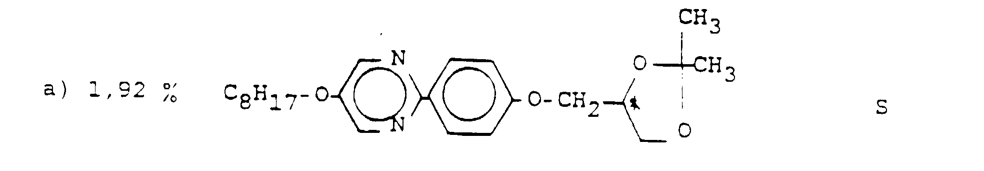

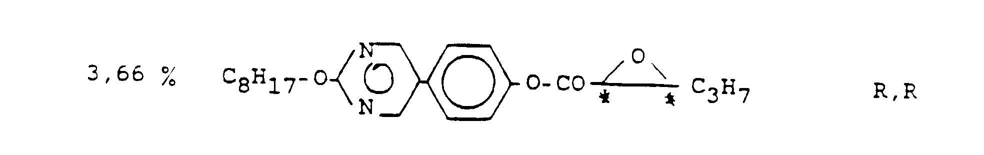

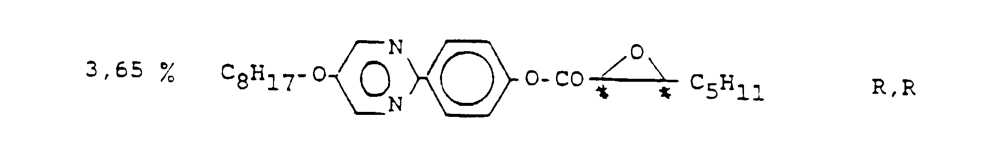

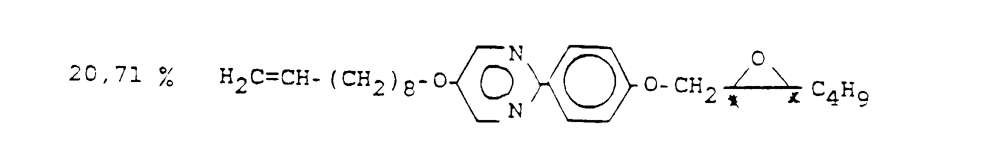

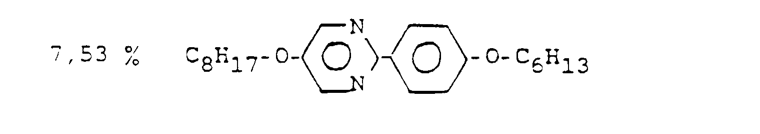

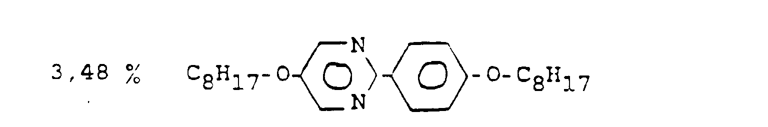

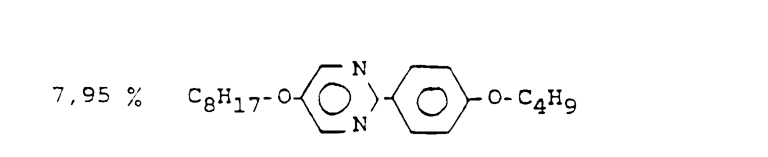

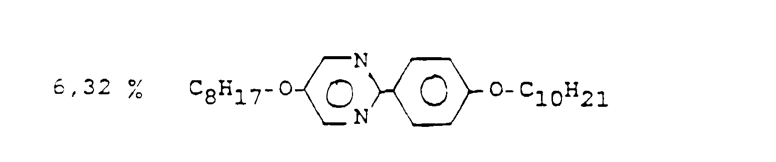

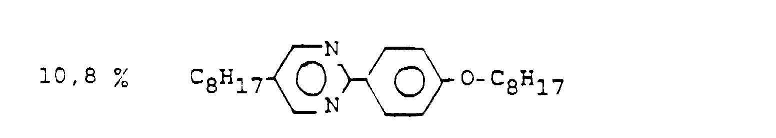

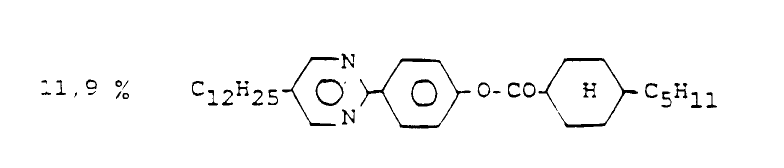

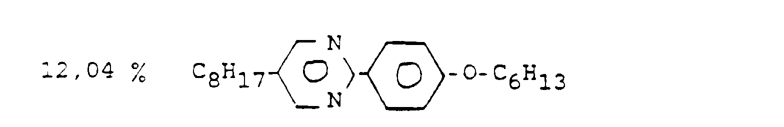

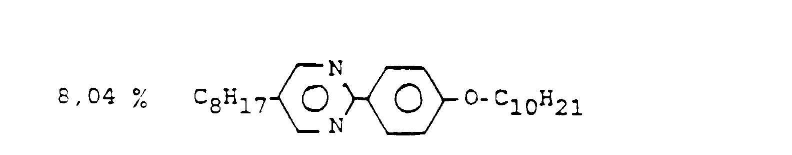

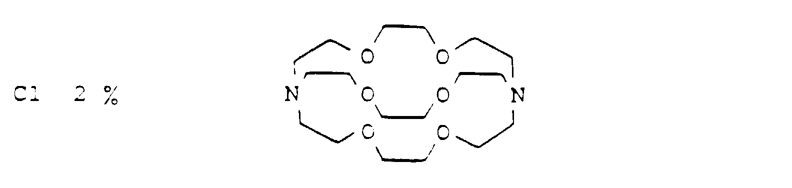

S_{C}* 56 ° S_{A}* 75 ° N 84 ° I

Ps = 35 nCb/cm²

Diese Mischung zeigt einen Schaltwinkel von 36,5 ° und kann durch einen bipolaren elektrischen Puls von 50 µs Breite und 10,4 V/µm Höhe in den gewünschten Schaltzustand gebracht werden.

### Vergleichsbeispiel

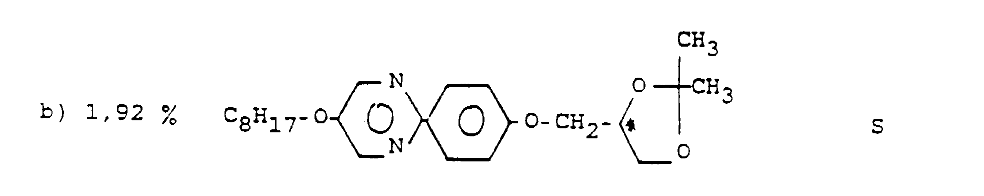

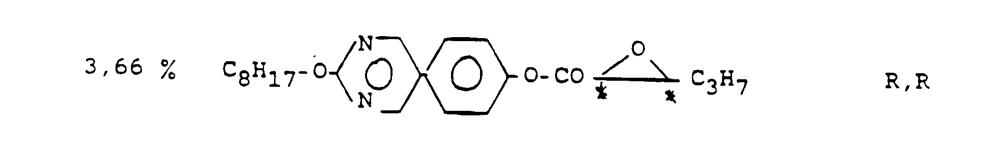

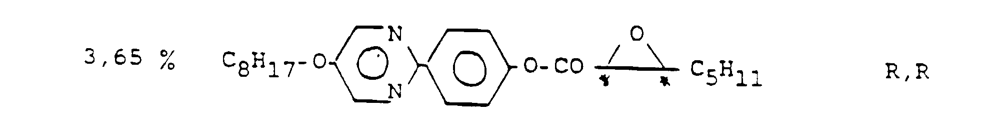

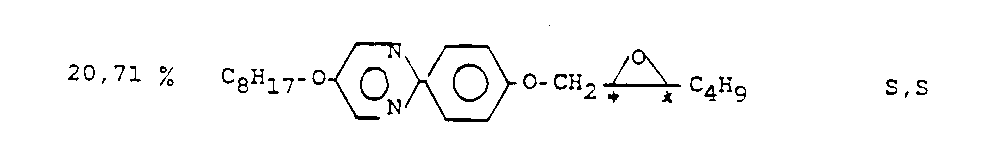

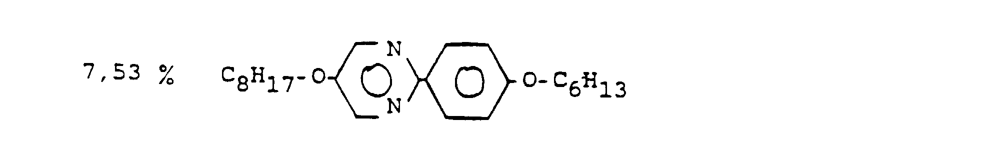

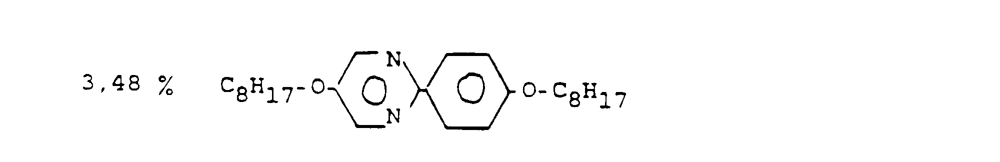

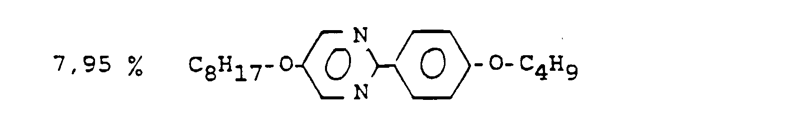

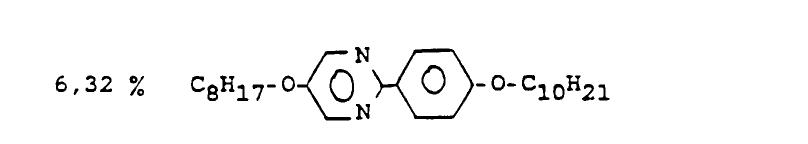

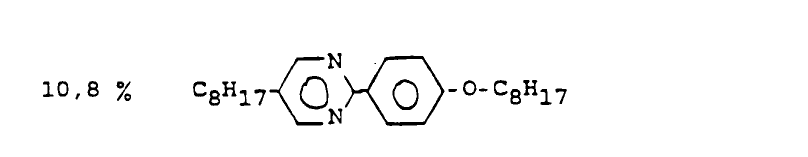

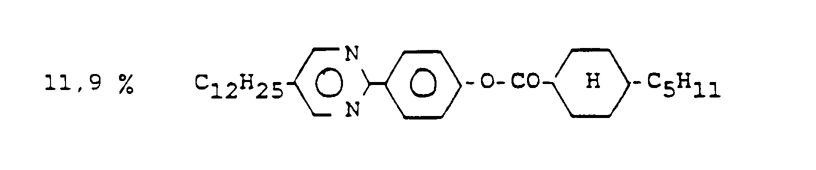

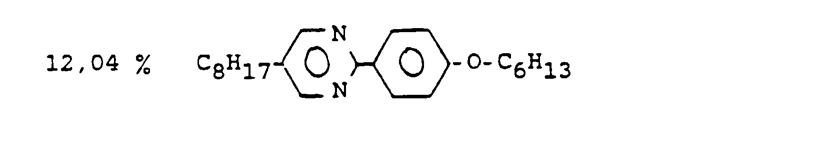

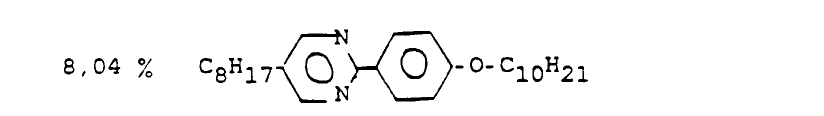

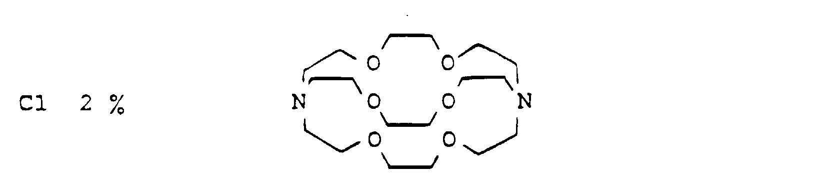

S_{C}* 59 ° S_{A}* 7° ° N 85 ° I

Ps = 44 nCb/cm²

Diese Mischung zeigt einen Schaltwinkel von 42,5 ° und kann durch einen bipolaren elektrischen Puls von 50 µs Breite und 19,6 V/µm Höhe in den gewünschten Schaltzustand gebracht werden.

## Patentansprüche

1. Chirale Verbindungen mit einem mesogenen aromatischen Baustein, einer Alkengruppe und einem chiralen Baustein mit einem heterocyclischen Dreiring, dadurch gekennzeichnet, daß in der allgemeinen Formel (I) 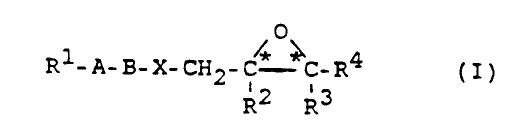 die Symbole folgende Bedeutung haben:
R¹ = geradkettiges oder verzweigtes (C₃-C₁₂)Alkenyl mit terminaler Doppelbindung, wobei eine oder zwei nicht-benachbarte CH₂-Gruppen durch O- und/oder S-Atome ersetzt sein können,
A, B = unabhängig voneinander Phenyl, Diazin-2,5-diyl oder Diazin-3,6-diyl, mit der Maßgabe, daß eine der Gruppen A oder B gleich Phenyl und die andere gleich Diazin ist,
X = O oder S, und
R²,R³,R⁴ = unabhängig voneinander H, geradkettiges (C₁-C₁₀)Alkyl oder verzweigtes (C₃-C₁₀)Alkyl, wobei R², R³ und R⁴ nicht gleichzeitig H sind.

2. Chirale Verbindungen nach Anspruch 1, dadurch gekennzeichnet, daß in der allgemeinen Formel (I) die Symbole folgende Bedeutung haben: R¹ = geradkettiges (C₅-C₁₁)Alkenyl, wobei eine CH₂-Gruppe durch ein O- oder S-Atom ersetzt sein kann, X=O, R²,R³=H und R⁴= geradkettiges oder verzweigtes (C₃-C₇)Alkyl.

3. Chirale Verbindungen nach Anspruch 1 oder 2, dadurch gekennzeichnet, daß A = Pyrimidin-2,5-diyl ist.

4. Verdrillbare Flüssigkristall-Phase mit einem Gehalt an mindestens einer chiralen Verbindung, dadurch gekennzeichnet, daß sie mindestens eine chirale Verbindung der allgemeinen Formel (I) nach Anspruch 1 enthält.

5. Verdrillbare Flüssigkristall-Phase nach Anspruch 4, dadurch gekennzeichnet, daß sie neben mindestens einer chiralen Verbindung der allgemeinen Formel (I) nach Anspruch 1 eine Phenylpyrimidin-Verbindung mit S_{c}-Phase enthält.

6. Flüssigkristall-Anzeigeelement enthaltend eine Flüssigkristall-Phase nach Anspruch 4.

7. Verwendung einer chiralen Verbindung gemäß Formel (I) nach Anspruch 1 zur Umwandlung einer geneigt-smektischen in eine ferroelektrische Flüssigkristall-Phase.

## Claims

1. A chiral compound containing a mesogenic aromatic unit, an alkene group and a chiral unit containing a heterocyclic three-membered ring, wherein, in the formula (I) 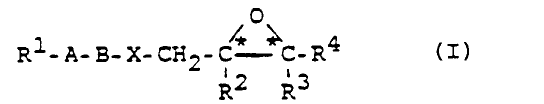 the symbols have the following meanings:
R¹ is straight-chain or branched (C₃-C₁₂)alkenyl containing a terminal double bond, it being possible for one or two non-adjacent CH₂ groups to be replaced by O and/or S atoms,
A and B, independently of one another, are phenyl, diazine-2,5-diyl or diazine-3,6-diyl, with the proviso that one of the groups A or B is phenyl and the other is diazine,
X is O or S, and
R², R³ and R⁴, independently of one another, are H, straight-chain (C₁-C₁₀)alkyl or branched (C₃-C₁₀)alkyl, R², R³ and R⁴ not simultaneously being H.

2. A chiral compound as claimed in claim 1, wherein, in the formula (I), the symbols have the following meanings: R¹ is straight-chain (C₅-C₁₁)alkenyl, it being possible for one CH₂ group to be replaced by an O or S atom, X is O, R² and R³ are H and R⁴ is straight-chain or branched (C₃-C₇)alkyl.

3. A chiral compound as claimed in claim 1 or 2, wherein A is pyrimidine-2,5-diyl.

4. A twistable liquid-crystal phase containing at least one chiral compound of the formula (I) as claimed in claim 1.

5. A twistable liquid-crystal phase as claimed in claim 4, which contains, in addition to at least one chiral compound of the formula (I) as claimed in claim 1, a phenylpyrimidine compound having an S_{c} phase.

6. A liquid-crystal display element containing a liquid-crystal phase as claimed in claim 4.

7. The use of a chiral compound of the formula (I) as claimed in claim 1 for converting a tilted smectic phase into a ferroelectric liquid-crystal phase.

## Revendications

1. Composés chiraux avec un constituant de base aromatique mésogène, un groupe alkyle et un constituant de base chiral avec un triple cycle hétérocyclique, caractérisé en ce que dans la formule générale (I) 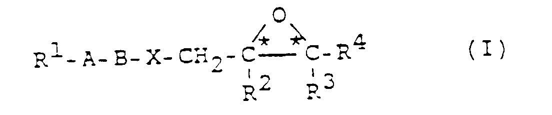 les symboles ont les significations suivantes :
R¹ représente un alcényle en C₃-C₁₂ linéaire ou ramifié avec une double liaison terminale, un ou deux groupe CH₂ voisins pouvant être remplacés par des atomes de O et/ou de S,
A, B indépendamment l'un de l'autre est phényle, diazine-2,5-diyle ou diazine-3,6-diyle, à la condition que l'un des groupes A ou B soit phényle et l'autre diazine,
X est O ou S, et
R², R³, R⁴, indépendamment les uns des autres, représentent H, alkyle en C₁-C₁₀ linéaire ou alkyle en C₁-C₁₀ ramifié, R², R³ et R⁴ n'étant pas simultanément H.

2. Composés chiraux selon la revendication 1, caractérisés en ce que dans la formule générale (I), les symboles ont la signification suivante : R¹ est alcényle en C₅-C₁₁ linéaire, un groupe CH₂ pouvant être remplacé par un atome de O ou de S, X est O, R², R³ est et R⁴ est alkyle en C₃-C₇ linéaire ou ramifié.

3. Composés chiraux selon la revendication 1 ou 2, caractérisés en ce que A est pyrimidine-2,5-diyle.

4. Phase de cristal liquide susceptible de torsion ayant une teneur en au moins un composé chiral, caractérisé en ce qu'elle contient un composé chiral de formule générale (I) selon la revendication 1.

5. Phase de cristal liquide susceptible de torsion selon la revendication 4, caractérisée en ce qu'elle contient outre au moins un composé chiral de formule générale (I) selon la revendication 1, un composé de phénylpyrimidine avec une phase Sc.

6. Elément d'affichage à cristal liquide contenant une phase de cristal liquide selon la revendication 4.

7. Utilisation d'un composé chiral selon la formule (I) selon la revendication 1, pour la conversion d'une phase de cristal liquide smectique inclinée en une phase de cristal liquide ferroélectrique.
